# EUROPEAN PATENT APPLICATION

(11) **EP 2 525 226 A1**
(43) Date of publication of application: **21.11.2012**
(21) Application number: 12168425.2
(22) Date of filing: 17.05.2012
(51) Int. Cl.: G01N 33/543, G01N 33/58, C12Q 1/68

(54) **Kits for detecting target material and methods of detecting target material using the kits**

(30) Priority: 17.05.2011 KR 20110046389
(71) Applicant: Samsung Electronics Co., Ltd., Suwon-si, Gyeonggi-do, 443-742 (KR); Postech Academy-Industry Foundation, Gyeongbuk-do (KR)
(72) Inventor: Im, Kyu-hyun, Gyeonggi-do (KR); Park, No-kyoung, Gyeonggi-do (KR); Park, Jong-jin, Gyeonggi-do (KR); Hur, Jae-hyun, Gyeonggi-do (KR); Park, Joon-hyuck, Gyeonsangbuk-do (KR); Kim, Sung-jee, Gyeonsangbuk-do (KR)
(74) Representative: Zijlstra, Robert Wiebo Johan

(57) **Abstract**

Kits for detecting a target material and methods of detecting a target material by using the kits are provided, the kits include a target material binding portion including a first molecule and a probe linked to the first molecule, and a target material detection portion including a plurality of nanoparticles each having a surface to which a compound having a zwitterion and a second molecule are linked. The first molecule is specifically bound to the second molecule in a pair.

## Description

### BACKGROUND

Example embodiments relate to kits for detecting a target material, and methods of detecting a target material using the same.

Avidin and biotin, like in the case of a reaction between an antibody and an antigen, bind very stably to each other to form a composite via an intermolecular interaction based on a non-covalent binding. Maximum four biotin molecules are linkable to one avidin molecule, and an interaction between avidin and biotin has the strongest bonding force from among currently known entities present in the natural system. However, when a fluorescent molecular sieve or an enzyme contacts avidin, a level of a non-specific binding to a material that is not intended to be detected, or to a substrate, is high. Thus, a signal-to-noise ratio becomes low, and it is difficult to embody (or realize) a high-sensitivity sensor. Accordingly, a target material may not be detected with high reproducibility and high sensitivity. Also, in the case of a fluorescent molecular sieve, when exposed to light (in particular, light in an ultraviolet region), fluorescent properties of the fluorescent molecular sieve decrease. In the case of an enzyme, according to a condition including (e.g., temperature or humidity), an activity thereof may easily decrease too. Thus, a detection kit employing a target material signal amplification method using the fluorescent molecular sieve or enzyme has poor long-term preservation properties. The detection kit employing a target material signal amplification method using the fluorescent molecular sieve or enzyme has limitations in self-diagnosing a cardiovascular disease that has a frequent acute onset, or a circulatory disease, in, for example, a personal environment (e.g., in a household), and in a medical treatment in field operations.

### SUMMARY OF THE INVENTION

Example embodiments relate to kits for detecting a target material, and methods of detecting a target material using the same.

Provided are kits for detecting a target material, wherein the kit includes a target material binding portion including a first molecule and a probe linked to the first molecule, and a target material detection portion including nanoparticles each having a surface to which a compound having a zwitterion and a second molecule are linked. The first molecule is specifically bound to the second molecule in a pair.

A quantum dot is hardly photo- bleached even when exposed to light of high energy for a long period of time. Thus, the quantum dot may emit a fluorescence signal more stably for a longer period of time than the fluorescent molecular sieve. Also, because a surface of the quantum dot is reformed to lower the level of non-specific binding, a signal-to-noise ratio relatively increases. Also, by introducing a zwitterion compound (which helps a surface charge of the overall compound to have charge characteristics that are resistant to change (i.e., characteristics that hardly change) according to surrounding conditions, and which has a substantially neutral surface compared to a surface of the quantum dot), a non-specific binding between the quantum dot and a material that is not intended to detect or a substrate may be minimized (or reduced).

By using such characteristics, a method of amplifying a target material detection signal quickly and easily can be realized.

According to example embodiments, the compound having a zwitterion is selected from the group consisting of an amino acid, bicine, tricine, a sulfamic acid, alkaloid, psilocybin, quinonoid, and betaine.

According to example embodiment, the second molecule may be linked to the surface of each of the nanoparticles via a linker binding to the surface of each of the nanoparticles. The linker may include a nanoparticle binding site and a nanoparticle non-binding site. The nanoparticle binding site may be selected from the group consisting of an organic acid comprising a hydroxyl group, a thiol group, or phenol group, and an organic base comprising pyridin, methylamine, imidazole, benzimidazole, histidine, phosphazene base, or a hydroxyl group, and the nanoparticle non-binding site is selected from the group consisting of an carboxylic acid and an azid group.

According to example embodiments, the nanoparticles may each include one selected from the group consisting of a quantum dot, gold, silver and iron oxide. The quantum dot may include at least one selected from the group consisting of Groups II to VI compound semiconductors, Groups III to V compound semiconductors, and Group IV compound semiconductors.

According to example embodiments, the pair of the first molecule and the second molecule may be one selected from the group consisting of a pair of a single stranded nucleic acid molecule and a single stranded nucleic acid molecule having a sequence complementary thereto, a pair of a double stranded DNA and a protein specifically binding thereto, and a pair of an antigen and an antibody. Alternatively, the pair of the first molecule and the second molecule may be one selected from the group consisting of a pair of streptavidin and biotin, and a pair of avidin and biotin.

According to example embodiments, the probe is specifically bound to the target material. The probe may be selected from the group consisting of a nucleic acid, a carbohydrate, an antibody, and a lipid.

According to example embodiments, the target material detection portion may further include a detectable label linked to the surface of each of the nanoparticles. The detectable label is selected from the group consisting of a colored bead, a coloring material, a fluorescent material, a phosphorescent material, electrically detectable molecule, and a material that supplies altered fluorescence-polarization or altered light-diffusion.

Provided are methods of detecting a target material by using the kits. The methods include contacting the target material binding portion of the kit and a target material to be detected to form a composite, contacting the composite and the target material detection portion, and detecting the target material.

According to example embodiments, the target material may be immobilized on a solid support. The solid support may be selected from the group consisting of a slide, a wafer, a bead, a membrane, and a plate. The target material may be selected from the group consisting of DNA, RNA, peptide nucleic acid (PNA), locked nucleic acid (LNA), a protein, a peptide, a carbohydrate, and a combination thereof.

According to example embodiments, the method may further include, after forming the composite, removing a non-bound target material binding portion prior to detection of the target material.

The method may further include, after contacting the composite and the target material detection portion, removing a non-bound target material detection portion to modify the composite prior to detection of the target material. After removing the non-bound target material detection portion, the method may include contacting the target material detection portion and the modified composite.

According to example embodiments, the detecting of the target material may include detecting a signal selected from the group consisting of a magnetic signal, an electrical signal, an emission signal, a scattering signal, and a radioactive signal.

According to example embodiments, provided is a detection kit including a target material binding portion including a first molecule and a probe linked to the first molecule, and at least one target material detection portion specifically bound to the target material binding portion via a second molecule having an affinity for the first molecule. The probe specifically binds to a target material. The at least one target material detection portion includes a signal-emitting particle that has a surface to which a compound having buffering characteristics and the second molecule are linked. The buffering characteristics prevent (or reduce) non-specific binding with the target material binding portion by imparting static charge characteristics (i.e., characteristics that are hardly changed) regardless of the surrounding conditions. The compound having buffering characteristics may have a substantially neutral surface compared to a surface of the signal-emitting particle.

According to example embodiments, the signal-emitting particle may emit a fluorescence signal.

The compound having buffering characteristics may include a molecule having both a positive electrical charge and a negative electrical charge.

According to example embodiments, provided is a detection kit including a target material binding portion including a first molecule and a probe linked to the first molecule, and at least one target material detection portion specifically bound to the target material binding portion via a second molecule having an affinity for the first molecule. The probe specifically binds to the target material. The at least one target material detection portion includes a plurality of photo-resistive particles each having a surface to which a compound having a molecule with a neutral surface charge and a second molecule are linked.

According to example embodiments, the molecule with a neutral surface charge may have both a positive electrical charge and a negative electrical charge.

The plurality of photo-resistive particles may be insensitive to ultraviolet light.

### BRIEF DESCRIPTION OF THE DRAWINGS

These and/or other aspects will become apparent and more readily appreciated from the following description of the embodiments, taken in conjunction with the accompanying drawings of which:
FIG. 1 is a schematic view of a target material detection portion according to example embodiments, wherein a zwitterion compound and streptavidin are linked to a surface of a quantum dot;
FIG. 2 is a graph for comparing absorbance (A) and emission spectrum (C) of a quantum dot with absorbance (B) and emission spectrum (D) of the zwitterion compound and streptavidin-linked quantum dot;
FIG. 3 shows results of a hydrodynamic size measured by dynamic light scattering with respect to a zwitterion compound and streptavidin-linked quantum dot (A), a biotin-linked quantum dot (B), a quantum dot before binding (C), a specific binding between the zwitterion compound and the streptavidin-linked quantum dot occurring due to an interaction therebetween (the number of each of the graphs is a measurement value of a vertex having the highest frequency);
FIG. 4 is a graph of a myoglobin detection signal amplification intensity according to a non-specific binding force at a surface of a quantum dot;
FIG. 5 is a schematic view illustrating a method of detecting myoglobin by using a target material detection portion and a target material binding portion according to example embodiments;
FIG. 6A is a graph of a fluorescence signal intensity with respect to a specific interaction between a zwitterion compound and streptavidin-linked quantum dot and a biotin-linked quantum dot according to a reaction time, and FIG. 6B is a graph of myoglobin detection fluorescence signal intensity according to a count of treatment with a biotin-linked quantum dot;

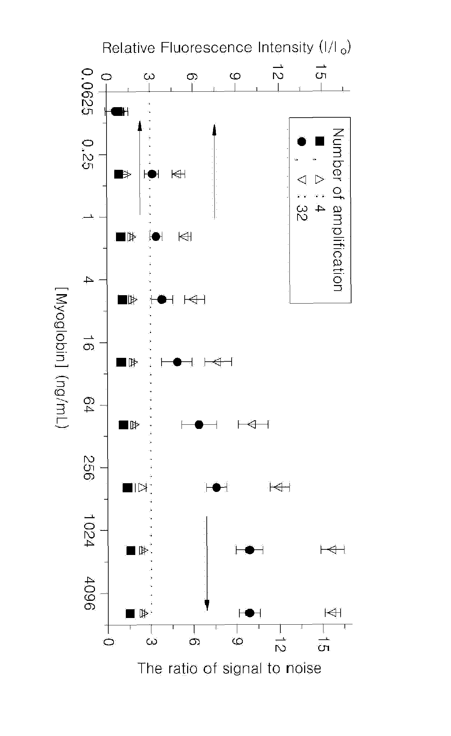

FIG. 7 is a detection signal intensity graph of myoglobin amplified due to a specific interaction between a zwitterion compound and streptavidin-linked quantum dot and a biotin-linked quantum dot, wherein from fluorescent microscope pictures of a detection substrate with respect to various myoglobin concentrations of 97.5 pg/mL to 1.60 µg/mL, results were quantified as a relative fluorescence intensity (respectively, number 32 (inverted triangle) and number 4 (triangle) amplified detection signals), and a signal-to-noise ratio (respectively, number 32 (circle) and number 4 (a regular square) amplified detection signals).

### DETAILED DESCRIPTION

Various example embodiments will now be described more fully with reference to the accompanying drawings in which some example embodiments are shown. However, specific structural and functional details disclosed herein are merely representative for purposes of describing example embodiments. Thus, the invention may be embodied in many alternate forms and should not be construed as limited to only example embodiments set forth herein. Therefore, it should be understood that there is no intent to limit example embodiments to the particular forms disclosed, but on the contrary, example embodiments are to cover all modifications, equivalents, and alternatives falling within the scope of the invention.

In the drawings, the thicknesses of layers and regions may be exaggerated for clarity, and like numbers refer to like elements throughout the description of the figures.

Although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of example embodiments. As used herein, the term "and/or" includes any and all combinations of one or more of the associated listed items.

It will be understood that, if an element is referred to as being "connected" or "coupled" to another element, it can be directly connected, or coupled, to the other element or intervening elements may be present. In contrast, if an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises," "comprising," "includes" and/or "including," if used herein, specify the presence of stated features, integers, steps, operations, elements and/or components, but do not preclude the presence or addition of one or more other features, integers, steps, operations, elements, components and/or groups thereof.

Spatially relative terms (e.g., "beneath," "below," "lower," "above," "upper" and the like) may be used herein for ease of description to describe one element or a relationship between a feature and another element or feature as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, for example, the term "below" can encompass both an orientation that is above, as well as, below. The device may be otherwise oriented (rotated 90 degrees or viewed or referenced at other orientations) and the spatially relative descriptors used herein should be interpreted accordingly.

Example embodiments are described herein with reference to cross-sectional illustrations that are schematic illustrations of idealized embodiments (and intermediate structures). As such, variations from the shapes of the illustrations as a result, for example, of manufacturing techniques and/or tolerances, may be expected. Thus, example embodiments should not be construed as limited to the particular shapes of regions illustrated herein but may include deviations in shapes that result, for example, from manufacturing. The regions illustrated in the figures are schematic in nature and their shapes do not necessarily illustrate the actual shape of a region of a device and do not limit the scope.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Example embodiments relate to kits for detecting a target material, and methods of detecting a target material using the same.

Example embodiments provide a kit for detecting a target material, wherein the kit includes a target material binding portion including a first molecule and a probe linked to the first molecule, and a target material detection portion including nanoparticles each having a surface to which a compound having a zwitterion and a second molecule are linked, wherein the first molecule is specifically bound to the second molecule in a pair.

The term "target material" refers to a material to be detected in a sample. The target material may be, for example, a biomolecule (e.g., a protein, a nucleic acid, a carbohydrate, or a lipid).

The kit includes the target material binding portion and the target material detection portion. The target material binding portion of the kit is a portion that directly binds to the target material, and the target material detection portion of the kit may detect presence of the target material via specific binding with the target material binding portion.

According to example embodiments, the target material binding portion may include the first molecule and the probe linked to the first molecule.

According to example embodiments, the first molecule may specifically bind to the second molecule included in the target material detection portion in a pair. The term "specifically binding" refers to an interaction between two or more molecules via a covalent bond or a non-covalent bond, and an example of the specifically binding is an immunological reaction between an antigen and an antibody via specifically binding. According to the definition described above, the pair of the first molecule and the second molecule may be, for example, selected from the group consisting of a pair of a single stranded nucleic acid molecule and a single stranded nucleic acid molecule having a sequence complementary thereto, a pair of a double stranded DNA and a protein specifically binding thereto, and a pair of an antigen and an antibody. In detail, the pair may be selected from the group consisting of a pair of streptavidin and biotin, a pair of avidin and horseradish peroxidase, a pair of streptavidin and alkaline phosphatase, and a pair of avidin and biotin, but is not limited thereto. Due to the specific binding between the first molecule and the second molecule, when a target material is detected, the target material binding portion including the first molecule may be located near the target material detection portion including the second molecule.

The term "probe" refers to a molecule that directly specifically binds to a target material during detection of the target material. Accordingly, depending on the target material, the probe may vary. For example, if the target material is an antigen protein, the probe may be an antibody that specifically binds to the antigen protein. According to example embodiments, the probe may be a molecule that specifically binds to a target material. The probe may be, for example, selected from the group consisting of a nucleic acid, a carbohydrate, an antibody, and a lipid, but is not limited thereto.

According to example embodiments, the target material detection portion may include nanoparticles each having a surface to which the compound having a zwitterion and the second molecule are linked.

The term "nanoparticle" refers to a particle that consists of materials having a diameter of about 1 to about 1000 nm, and the nanoparticle may be a metallic nanoparticle or a non-metallic nanoparticle. According to example embodiments, the metallic nanoparticle may be selected from the group consisting of gold, silver, copper, aluminum, nickel, palladium, plutonium, a magnetic iron, and an oxide thereof, but is not limited thereto. The non-metallic nanoparticle may be selected from the group consisting of silica, polystyrene, latex, and an acrylate-based material, but is not limited thereto. Also, the nanoparticle may be a quantum dot composite including one or more selected from the group consisting of Groups II to VI compound semiconductors, Groups III to V compound semiconductors, and Group IV compound semiconductors, of the Periodic Table.

The term "zwitterion" refers to a molecule that includes a positive charge and a negative charge at different positions in the same molecule. When the compound having a zwitterion is ionized in an aqueous solution, the compound has a positive charge and a negative electrical charge. The compound having a zwitterion is linked to a surface of the target material detection portion to prevent non-specific binding with a target material binding portion according to example embodiments. According to example embodiments, the compound having a zwitterion may be, for example, selected from the group consisting of an amino acid, bicine, tricine, a sulfamic acid, alkaloid, psilocybin, quinonoid, and betaine, but is not limited thereto.

Also, the second molecule may specifically bind to the first molecule included in the target material binding portion in a pair. The pair of the first molecule and the second molecule has been described above. Due to the specific binding between the second molecule and the first molecule, during the detection of a target material, the target material binding portion including the first molecule may be positioned near the target material detection portion including the second molecule.

According to embodiments, the second molecule may be linked via a linker binding to a surface of each of the nanoparticles. The linker may be any one of various compounds used as a linker in the art, and may be appropriately selected according to the functional group(s)present in the second molecule. For example, the linker may be selected from the group consisting of an organic acid including a hydroxyl group, a thiol group, or phenol group, and an organic base including pyridin, methylamine, imidazole, benzimidazole, histidine, phosphazene base, or a hydroxyl group, but is not limited thereto.

According to example embodiments, the target material detection portion may further include a detectable label linked to the surface of each of the nanoparticle.

The term "detectable label" may be an atom or molecule that specifically detects a molecule including a label among the same kind of molecules having no label. The detectable label may include, for example, a colored bead, an antigen crystal, an enzyme, a hybridizable nucleic acid, a coloring material, a fluorescent material, a phosphorescent material, an electrically detectable molecule, or material that supplies altered fluorescence-polarization or altered light-diffusion. Also, the label may include a radioactive isotrope (e.g., ³²P and ³⁵S), a chemiluminescent compound, a labeled binding protein, a heavy metal atom, and a spectroscopic marker (e.g., die, or a magnetic labeling material).

Other example embodiments provide a method of detecting a target material using the kit, including contacting the target material binding portion of the kit and a target material to be detected to form a composite; contacting the composite and the target material detection portion of the kit; and detecting the target material.

The target material detection method will now be described in detail.

First, the method may include contacting the target material binding portion of the kit and a target material to be detected.

According to example embodiments, the target material may be immobilized on a solid support. For example, the solid support may be selected from the group consisting of a slide, a wafer, a bead, a membrane, and a plate, but is not limited thereto. According to example embodiments, the target material may differ according to a target material to be detected, and non-limiting examples thereof are a nucleic acid (e.g., DNA, RNA, peptide nucleic acid (PNA), or locked nucleic acid (LNA)), a protein, a peptide, a carbohydrate, and a combination thereof.

The contacting may be performed in a buffer solution widely known in the art. That is, the contacting may be performed in a condition that molecules present in the target material and the target material binding portion interact in a stable state. Also, the contacting may be performed by, for example, directly adding a buffer solution including the target material binding portion to the target material immobilized on the solid support.

Through the contacting, the probe binding to the surface of the target material binding portion may specifically binds to the target material, and accordingly, the target material binding portion binds to the target material while the first molecule is exposed.

According to example embodiments, the method may further include, after forming the composite, removing a non-bound target material binding portion. When removing the non-bound target material binding portion, the target material binding portion may be removed by adding excess washing solution that is widely known in the art, for example, distilled water, alcohol, etc.

Then, the method includes contacting the composite and the target material detection portion of the kit.

The contacting, like during formation of the composite, may be performed in a buffer solution widely known in the art. That is, the contacting may be performed in a condition that molecules present in the target material, the target material binding portion, and the target material detection portion interact in a stable state. Also, the contacting may be performed by, for example, directly adding a buffer solution including the target material detection portion to result of the operation a).

As a result of forming the composite, the target material specifically binds to the probe of the target material binding portion, and the first molecule linked to the probe is exposed. Accordingly, when the target material detection portion is brought into contact therewith, the second molecule linked to the surface of the target material detection portion specifically binds to the first molecule, and in this case, the zwitterion compound present at the surface of the target material aids (or helps) a surface charge to have such charge characteristics that are hardly changed according to surrounding conditions, and to have almost neutrality, and thus, non-specific binding between the solid support and the target material detection portion may be preventable.

According to example embodiments, the method further includes, after contacting the composite and the target material detection portion, removing a non-bound target material detection portion to modify the composite, before detecting the target material.

When removing the non-bound target material detection portion, the target material binding portion may be removed by adding excess washing solution that is widely known in the art of the present invention, for example, distilled water, alcohol, etc.

According to example embodiments, the method further comprises, after removing the non-bound target material detection portion, contacting the target material detection portion of the kit and the modified composite. The method may further include repeatedly performing (e.g., one to 24 times) a cycle including contact of the composite and the target material detection portion, removal of the non-bound target material detection portion, and contact of the target material detection portion of the kit and the modified composite.

This operation means repeatedly contacting the target material detection portion that has contact with the composite when contacting the composite and the target material detection portion. The first molecule included in the target material binding portion may specifically binds to a plurality of second molecules depending on the molecules. Accordingly, if the contacting of the target material detection portion including the second molecule is repeatedly performed until the number of second molecules linkable to the first molecule is saturated, the target material detection portion may specifically bind to the target material binding portion. If a plurality of target material binding portions and target material detection portions are present adjacent to each other due to the specific binding, a signal detectable by the target material detection portion may be amplified.

The method further includes detecting the target material.

According to example embodiments, when detecting the target material, a signal selected from the group consisting of a magnetic signal, an electrical signal, an emission signal, a scattering signal, and a radioactive signal may be detected. The signal may be, for example, selected from the group consisting of a magnetic signal, an electrical signal, an emission signal, such as, a fluorescent or a Raman signal, a scattering signal, and a radioactive signal, which are generated from the detectable label. A detailed example of the detection signal is the same as presented with respect to the detectable label.

Example embodiments will now be described in further detail with reference to the following examples. These examples are for illustrative purpose only and are not intended to limit the scope of the one or more embodiments.

### Example 1: Synthesis of target material detection portion of which non-specific binding is minimized

Nanoparticles each having a core/shell structure of CdSe/CdZnS synthesized by using a high-temperature thermal decomposition process in which cadmium (Cd), selenium (Se), zinc (Zn), and sulfur(S) precursors were rapidly added to a high-temperature and nitrogen gas (N₂)-saturated solvent including octadecene and oleylamine were dispersed in chloroform. The nanoparticles were used as quantum dots. Also, an aqueous solution in which a previously synthesized zwitterion compound represented by Formula I below and a compound including a carboxylic group represented by Formula II below had been excessively dissolved was mixed with a chloroform solution including purified quantum dots, and the mixture was stirred at room temperature.

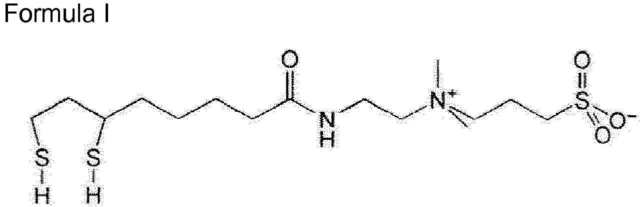

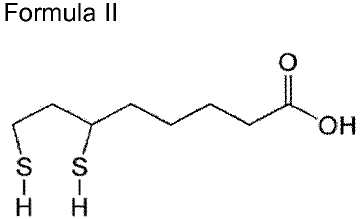

In this regard, an organic molecular ligand (e.g., trioctylphosphine, trioctylphosphine oxide, oleylamine, an oleic acid, or the like), present at a surface of each of the quantum dots was simultaneously substituted with the zwitterion compound and the compound including a carboxylic group, so that the quantum dots were moved to an aqueous solution layer and dispersed therein. Then, a chloroform layer was isolated, and only the aqueous solution layer was dialyzed to remove the residual ligand. Then, the quantum dots simultaneously surface-substituted with the carboxylic acid and the zwitterion compound was treated with a carbodiimide-based binding reagent for aiding a covalent bond, and 125 µL of a streptavidin solution was added thereto in such a way that 2.5 mg/mL of streptavidin solution corresponded to 1 nmol of a quantum dot, thereby binding streptavidin to the carboxylic group. Thus, a quantum dot (constitutes a target material detection portion) having a surface to which the zwitterion compound and streptavidin were linked.

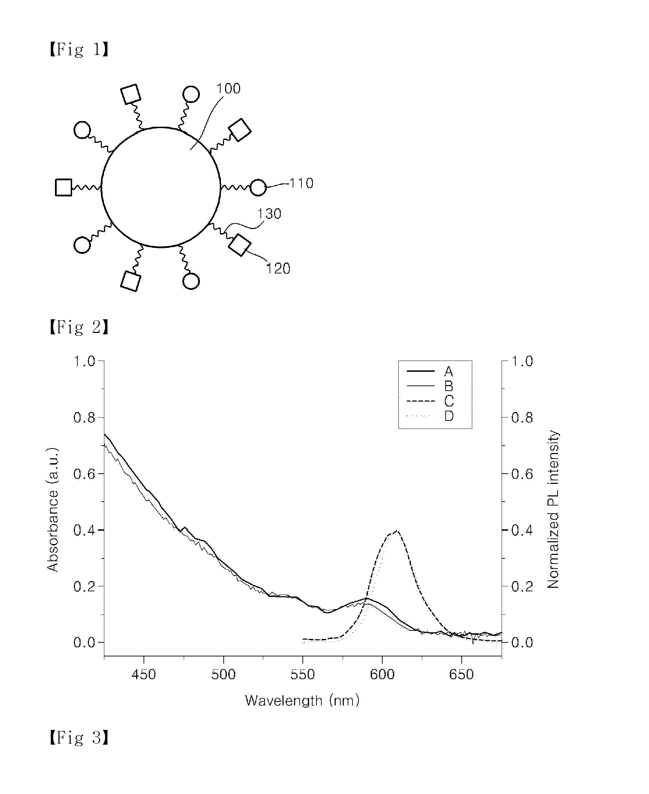

FIG. 1 is a schematic view of the target material detection portion according to example embodiments.

Referring to FIG. 1, the target material detection portion includes a quantum dot 100 having a surface to which a zwitterion compound 110 and a streptavidin 120 as an example of a second molecule are linked via an organic molecule ligand 130.

FIG. 2 is a graph for comparing absorbance (A) and emission spectrum (C) of a quantum dot with absorbance (B) and emission spectrum (D) of the zwitterion compound and streptavidin-linked quantum dot.

As illustrated in FIG. 2, it was confirmed that although the zwitterion compound and streptavidin were linked to the target material detection portion, absorption and emission characteristics of quantum dots constituting the target material detection portion were maintained well compared to a control group to which the zwitterion compound and streptavidin were not linked.

### Example 2: Confirmation of self assembling due to specific binding between the streptavidin of the target material detection portion and biotin

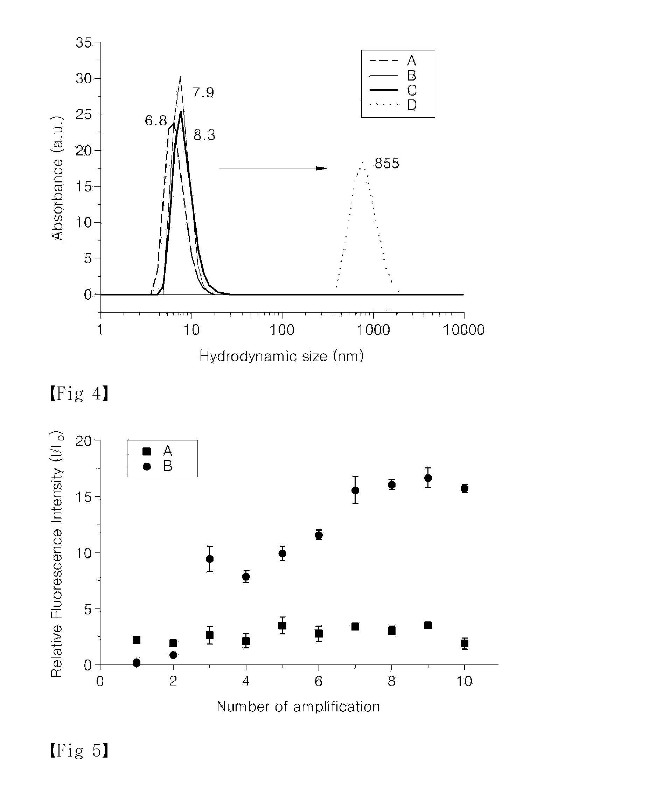

After the quantum dot simultaneously surface-substituted with the carboxylic group and the zwitterion compound was treated with the carbodiimide-based binding reagent for aiding a covalent bond, a biotin to which a primary amine was linked was added thereto, thereby preparing a biotin-linked quantum dot. In this regard, 36 µL of a biotin solution was used in such a way that 1 mg/mL of the biotin solution corresponded to 1 nmol quantum dot. Then, the biotin-linked quantum dot was mixed with the target material detection portion prepared according to Example 1, and then left to sit for 30 minutes and then, a hydrodynamic size thereof was measured by dynamic light scattering. Results thereof are shown in FIG. 3.

FIG. 3 shows results of a hydrodynamic size measured by dynamic light scattering with respect to a zwitterion compound and streptavidin-linked quantum dot (A), a biotin-linked quantum dot (B), a quantum dot before binding (C), a specific binding between the zwitterion compound and the streptavidin-linked quantum dot occurring due to an interaction therebetween (the number of each of the graphs is a measurement value of a vertex having the highest frequency).

Referring to FIG. 3, a hydrodynamic size (A of FIG. 3) of a quantum dot surface-substituted with only the zwitterion compound and the compound including the carboxylic group without addition of the streptavidin unlike in Example 1 was less than the size of the quantum dot to which the target material detection portion or biotin was linked (B or C of FIG. 3) by about 1 to about 1.5 nm. This result may be due to the fact that a composition having a particle size increased due to streptavidin and biotin specifically binding to each other at a surface of the quantum dot moves more slowly than in the case when streptavidin was not introduced. Also, a hydrodynamic size (D of FIG. 3) of a sample in which the target material detection portion was mixed with a biotin-linked quantum dot was about 1000 nm. This result may be due to the fact that when a plurality of target material detection portions contact a plurality of biotin-linked quantum dots, they continuously provide a specific binding site to each other, thereby aggregating composites.

### Example 3: Confirmation of amplification of fluorescence signal of biotin-linked quantum dots through interaction between streptavidin and biotin

The target material detection portion prepared according to Example 1 was brought into contact with a biotin-linked quantum dot while an amount of the biotin-linked quantum dot was gradually increased. As a result, specific binding between streptavidin present in the target material detection portion and the biotin-linked quantum dot was actively performed. Because four (4) biotins are linkable to one streptavidin, the greater the amount of the biotin-linked quantum dot, the greater intensity a fluorescence signal of quantum dot has.

FIG. 4 is a graph of a myoglobin detection signal amplification intensity according to a non-specific binding force at a surface of a quantum dot.

As illustrated in FIG. 4A), if non-specific binding was not minimized, that is, a quantum dot that was not surface-treated with a zwitterion compound, when the amount of the biotin-linked quantum dot increased, the non-specific binding substantially increased. Thus, a noise signal increased too (B of FIG. 4). However, in the case of a quantum dot surface-treated with a zwitterion compound, even when a biotin-linked quantum dot was treated, a noise signal was relatively very low, and the greater the amount of the biotin-linked quantum dot, the greater intensity the fluorescence signal. Thus, when the amount of the biotin-linked quantum dot reached a certain level, the intensity of the fluorescence signal had a maximum value (A of FIG. 4).

### Example 4: Confirmation of reaction speed with respect to self assembling of target material detection portion and target material binding portion due to specific binding between streptavidin and biotin, and target material detection signal amplification test

As described in Example 3, it was confirmed that a fluorescence signal was effectively enhanced by linking a zwitterion compound to a surface of a nanoparticle so as to decrease non-specific binding. This result was actually confirmed by detecting myoglobin as a target material.

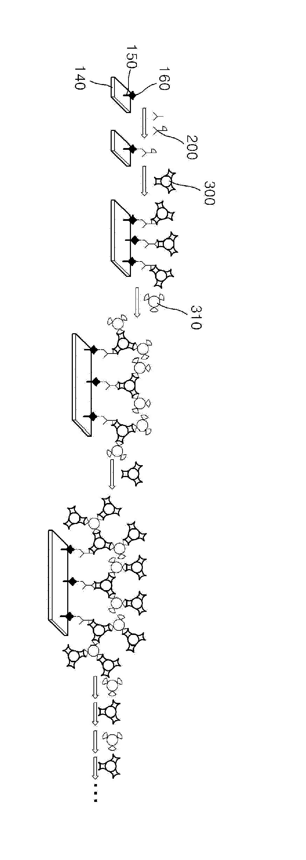

FIG. 5 is a schematic view illustrating a method of detecting myoglobin by using a target material detection portion and a target material binding portion according to example embodiments.

Referring to FIG. 5, a myoglobin antibody 150 was immobilized on a substrate 140, and a myoglobin 160 was brought into contact with the myoglobin antibody 150 on the substrate, and then a biotin-linked myoglobin antibody target material binding portion 200 was treated thereon, thereby exposing biotin to a surface of the substrate 140. Then, a nanoparticle target material detection portion 300 having a surface to which streptavidin and a zwitterion compound were linked and a nanoparticle target material detection portion 310 having a surface to which biotin and a zwitterion compound were repeatedly treated thereon. As a result, as illustrated in FIG. 5, according to a treatment count of a target material detection portion, a plurality of target material detection portions were linked in a dendrimer form to one target material binding portion. Accordingly, a fluorescence signal were amplified from quantum dots.

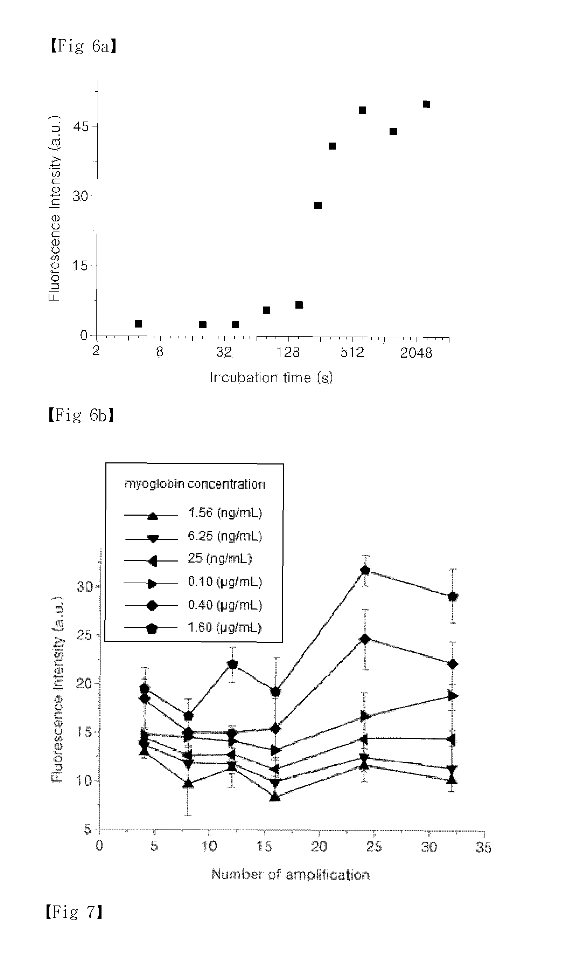

In this case, it was confirmed how fast a streptavidin and zwitterion compound-linked quantum dot and a biotin-linked quantum dot specifically bind to each other. As illustrated in FIG. 5, a contact time between a streptavidin and zwitterion compound-linked quantum dot and a biotin-linked quantum dot was variously controlled while biotin was exposed on the substrate 140 to obtain results of FIG. 6.

FIG. 6A is a graph of a fluorescence signal intensity with respect to a specific interaction between a zwitterion compound and streptavidin-linked quantum dot and a biotin-linked quantum dot according to a reaction time, and FIG. 6B is a graph of myoglobin detection fluorescence signal intensity according to a count of treatment with a biotin-linked quantum dot.

Referring to FIG. 6A, less than three minutes after the treatment with the streptavidin and zwitterion compound-linked quantum dot and the biotin-linked quantum dot, the fluorescence signal was substantially increased. 4 minutes after the treatment, the intensity of the fluorescent signal converged to its maximum value. From this result, it was confirmed that the binding between the streptavidin and zwitterion compound-linked quantum dot and the biotin-linked quantum dot occurs very fast, and after a certain period of time, a very stable state is obtainable.

Also, with respect to various myoglobin concentrations, the number of maximum signal amplifications when the streptavidin and zwitterion compound-linked quantum dot and the biotin-linked quantum dot were repeatedly treated was evaluated. As illustrated in FIG. 6B, a fluorescent detection signal was measured during 32 times of the treatments, and in this regard, the treatment time of the streptavidin and zwitterion compound-linked quantum dot and the biotin-linked quantum dot was 4 minutes for each. As a result, the greater the treatment count, the greater intensity the fluorescence signal has. Thus, the fluorescence signal overall increased, but after the 24^{th} treatment (i.e., after 24 times of signal amplifications), the fluorescence signal converged to its maximum value.

### Example 5: High-sensitive target material detection method according to repeated treatment with target material detection portion and target material binding portion

Based on the results obtained from Examples 2 to 4, it was confirmed that a pair of a streptavidin and zwitterion compound-linked quantum dot and a biotin-linked quantum dot was used in amplifying a detection signal of a target material (for example, myoglobin). This was embodied as a detection method that has very high sensitivity than a conventionally known signal amplification method.

FIG. 7 is a detection signal intensity graph of myoglobin amplified due to a specific interaction between a zwitterion compound and streptavidin-linked quantum dot and a biotin-linked quantum dot, wherein from fluorescent microscope pictures of a detection substrate with respect to various myoglobin concentrations of 97.5 pg/mL to 1.60 µg/mL, results were quantified as a relative fluorescence intensity (respectively, number 32 (inverted triangle) and number 4 (triangle) amplified detection signals), and a signal-to-noise ratio (respectively, number 32 (circle) and number 4 (a regular square) amplified detection signals).

Referring to FIG. 7, based on a fluorescent microscope picture of a substrate of which a fluorescence signal was amplified, it was confirmed that the greater the myoglobin concentration, the greater the relative fluorescent intensity and the signal-to-noise ratio. From this result, mostly, it was confirmed that the greater the myoglobin concentration, the greater intensity the fluorescence signal. However, when the myoglobin concentration reached 1000 ng/mL or more, the substrate coated with an antibody reached its own detection limit, and thus, the fluorescence signal converged to a maximum value. Also, with respect to a sample concentration in which the signal-to-noise ratio was 3 or more, a detection limitation of this system was about 390 pg/mL. The detection limitation is about 13 times lower than a typical myoglobin detection method based on an enzyme. Also, in consideration that typically, the enzyme based myoglobin detection method requires a detection time of about 180 minutes, a target material detection method according to example embodiments may be used to detect myoglobin with high sensitivity while a detection time thereof is about 120 or less, much shorter than the 180 minutes. Also, a target material detection method according to example embodiments is used to detect a target material without use of an enzyme.

As described above, a target material is efficiently detected by using kits for detecting a target material and methods of detecting a target material using the kits according to example embodiments.

It should be understood that the exemplary embodiments described therein should be considered in a descriptive sense only and not for purposes of limitation. Descriptions of features or aspects within each embodiment should typically be considered as available for other similar features or aspects in other embodiments.

## Claims

1. A kit for detecting a target material, the kit comprising:
a target material binding portion including a first molecule and a probe linked to the first molecule; and
a target material detection portion selected from:
a) a target material detection portion including a plurality of nanoparticles each having a surface to which a compound having a zwitterion and a second molecule are linked,
wherein the first molecule is specifically bound to the second molecule in a pair;
b) a target material detection portion specifically bound to the target material binding portion via a second molecule having an affinity for the first molecule,
wherein the at least one target material detection portion includes a signal-emitting particle that has a surface to which a compound having buffering characteristics and the second molecule are linked; and
c) a target material detection portion specifically bound to the target material binding portion via a second molecule having an affinity for the first molecule,
wherein the at least one target material detection portion includes a plurality of photo-resistive particles each having a surface to which a compound having a molecule with a neutral surface charge and a second molecule are linked.

2. The kit of claim 1, wherein the compound having a zwitterion is selected from the group consisting of an amino acid, bicine, tricine, a sulfamic acid, alkaloid, psilocybin, quinonoid, and betaine.

3. The kit of claim 1 or 2, wherein the second molecule is linked to the surface of each of the nanoparticles via a linker binding to the surface of each of the nanoparticles.

4. The kit of claim 3, wherein the linker includes a nanoparticle binding site and a nanoparticle non-binding site, wherein the nanoparticle binding site is selected from the group consisting of an organic acid comprising a hydroxyl group, a thiol group, or phenol group, and an organic base comprising pyridin, methylamine, imidazole, benzimidazole, histidine, phosphazene base, or a hydroxyl group, and the nanoparticle non-binding site is selected from the group consisting of an carboxylic acid and an azid group.

5. The kit of any of claims 1-4, wherein the nanoparticles each include one selected from the group consisting of a quantum dot, gold, silver and iron oxide, and the quantum dot includes at least one selected from the group consisting of Groups IIto VI compound semiconductors, Groups III to V compound semiconductors, and Group IV compound semiconductors.

6. The kit of any of claims 1-5, wherein the pair of the first molecule and the second molecule is one selected from the group consisting of a pair of a single stranded nucleic acid molecule and a single stranded nucleic acid molecule having a sequence complementary thereto, a pair of a double stranded DNA and a protein specifically binding thereto, a pair of an antigen and an antibody a pair of streptavidin and biotin, and a pair of avidin and biotin.

7. The kit of any of claims 1-6, wherein the probe is specifically bound to the target material.

8. The kit of any of claims 1-7, wherein the probe is selected from the group consisting of a nucleic acid, a carbohydrate, an antibody, and a lipid.

9. The detection kit according to claim 1, wherein the compound having buffering characteristics includes a molecule having both a positive electrical charge and a negative electrical charge and/or has a neutral surface compared to a surface of the signal-emitting particle.

10. A method of detecting a target material, the method comprising:
contacting the target material binding portion of the kit according to any of claims 1-9 and a target material to be detected to form a composite;
contacting the composite and the target material detection portion; and
detecting the target material.

11. The method of claim 10, wherein the target material is immobilized on a solid support.

12. The method of claim 11, wherein the solid support is selected from the group consisting of a slide, a wafer, a bead, a membrane, and a plate.

13. The method of any of claims 10-12, wherein the target material is selected from the group consisting of DNA, RNA, peptide nucleic acid (PNA), locked nucleic acid (LNA), a protein, a peptide, a carbohydrate, and a combination thereof.

14. The method of any of claims 10-13, wherein the method further comprises, after forming the composite, removing a non-bound target material binding portion prior to detection of the target material, wherein removing a non-bound target material detection portion optionally modifies the composite prior to detection of the target material.

15. The method of any of claims 10-14, wherein the detecting of the target material includes detecting a signal selected from the group consisting of a magnetic signal, an electrical signal, an emission signal, a scattering signal, and a radioactive signal.
